# EUROPEAN PATENT SPECIFICATION

(11) **EP 1 469 867 B1**
(45) Date of publication and mention of the grant of the patent: **12.08.2009**
(21) Application number: 02779021.1
(22) Date of filing: 27.11.2002
(51) Int. Cl.: A61K 33/24, A61P 1/00, A61P 43/00

(54) **METHOD OF TREATING HALITOSIS**
VERFAHREN ZUR BEHANDLUNG VON HALITOSE
METHODE DE TRAITEMENT DE L'HALITOSE

(30) Priority: 28.11.2001 AU PR916101
(43) Date of publication of application: 27.10.2004
(73) Proprietor: Bolin, Terry Dorcen, Randwick, NSW 2031 (AU)
(72) Inventor: Bolin, Terry Dorcen, Randwick, NSW 2031 (AU)
(74) Representative: Ryan, Anne Mary
(86) International application number: PCT/AU2002/001604
(87) International publication number: WO 2003/045403

(56) References cited:
- WO-A-97/07757
- WO-A-98/19669
- US-A- 5 834 002
- TIOMMY E ET AL: "HALITOSIS AND HELICOBACTER PYLORI A POSSIBLE LINK ?" JOURNAL OF CLINICAL GASTROENTEROLOGY, RAVEN PRESS LTD., NEW YORK, NY, US, vol. 15, no. 3, 15 October 1992 (1992-10-15), pages 236-237, XP001012914 ISSN: 0192-0790
- IERARDI E, AMORUSO A, LA NOTTE T, FRANCAVILLA R, CASTELLANETA S, MARRAZZA E, MONNO R A, FRANCAVILLA A: "Halitosis and Helicobacter pylori. A possible relationship" DIGESTIVE DISEASES AND SCIENCES, vol. 43, no. 12, December 1998 (1998-12), pages 2733-2737, XP008051131

## Description

### Technical Field

The present invention relates to a method of treating halitosis.

### Background of the Invention

Halitosis, or malodorous breath, is a common condition for which the exact pathophysiological mechanism remains unclear. It is generally assumed that malodorous compounds originate in the mouth.

The amount of odorous gas expelled in the breath varies with the dietary intake. Higher levels of odorous gas is usually produced following ingestion of increased levels of added sulfite in foods such as delicatessen meats, dried fruits, cask white wine, as well as being a normal component of many dietary nutrients. It is believed that the intensity of malodorous breath, or halitosis, is associated with intra-oral levels of volatile sulfide.

In addition to diet, halitosis is commonly thought to be related to conditions such as periodontal disease, stomatitis, rhinitis, pharyngitis, chronic tonsillitis and pharyngeal tumours. There is also an apparent association between halitosis and *Helicobacter pylori* infection in the gut. However, investigation and treatment of these conditions as causative factors of halitosis is usually unrewarding or inadequate.

Methods known in the art for treating halitosis are varied and generally have varying degrees of success. Most methods are directed to the oral cavity and include dental hygiene, periodontal care regimes, the use of oral mouth washes and perfumed drops. Such methods generally aim to mask the malodour, or reduce generation of malodorous gases of oral origin. Other methods of treating halitosis involve treatment of bacterial infection, for example, by treating conditions such as ulcers, periodontal disease, etc in the oral cavity, and/or eradication of *H. pylori* infection in the gut during treatment of peptic ulcers, eg, using triple therapy (a treatment regime comprising administration of omeprazole, clarithromycin and amoxycillin). However, after the above methods of treatment, halitosis may persist or re-occur upon cessation of treatment.

Suarez *et al. (*F. Suarez, J. Springfield, J. Fume and M. Levitt. Am. J. Physiol., 1999*, (Gastrointestin LiverPhysiol. 39*):*G425-G430*) have investigated the differentiation of mouth versus gut as the site of origin of odoriferous breath gases after garlic ingestion. They concluded that breath odour after garlic ingestion initially originates from the mouth and subsequently from the gut.

There is a need for effective treatment of halitosis in vertebrates. In particular, there is a need for treating halitosis which is not associated with *H. pylori* infection.

WO 97/07757 describes a method of treating halitosis by eradication of *Helicobacter pylori, Campylobacter rectus* and *Treponema denticola* bacteria causing halitosis in the oral cavity by administration of bismuth compounds.

WO 98/19669 discloses a chewing gum containing bismuth-containing compounds for the eradication of *Helicobacter pylori* for the treatment of ulcers and halitosis.

### Summary of the Invention

According to the invention there is provided use of a therapeutically effective amount of a bismuth salt or bismuth containing composition in the manufacture of a medicament for treating halitosis in a vertebrate in need of said treatment wherein said halitosis is associated with sulfite-reducing bacteria in the gut.

With reference to the above embodiment of the invention, typically the vertebrate is a mammal. More typically, the vertebrate is selected from the group consisting of human, non-human primate, murine, bovine, ovine, equine, caprine, leporine, avian, feline and canine. Still more typically, the vertebrate is human, or a farm or companion animal. Even more typically, the vertebrate is human.

Typically, the vertebrate has a sulfite-reducing bacterial population in the gut More typically, the vertebrate does not suffer from *Helicobacter pylori* infection.

Typically, a bismuth containing composition comprises a bismuth salt, together with a pharmaceutically acceptable carrier, adjuvant or diluent.

Typically the bismuth salt is selected from the group consisting of bismuth subcitrate, bismuth aluminate, bismuth oxide, bismuth salicylate, bismuth subgallate, bismuth stannate, bismuth phosphate, bismuth tribromphenate, bismuth subcarbonate, bismuth subnitrate, bismuth sodium tartrate, and mixtures thereof. More typically, the bismuth salt is selected from the group consisting of bismuth salicylate and bismuth subcitrate.

Typically, the bismuth containing composition is selected from the group consisting of Denol™ (active ingredient bismuth subcitrate) and Pepto-bismol^{®} (active ingredient bismuth subcitrate).

Typically, the bismuth salt or bismuth containing composition is administered orally.

### Definitions

In the context of this specification, the term "comprising" means "including principally, but not necessarily solely". Furthermore, variations of the word "comprising", such as comprise and "comprises", have correspondingly varied meanings.

The term "therapeutically effective amount" as used herein, includes within its meaning a non-toxic but sufficient amount of a bismuth salt or bismuth containing composition to provide the desired therapeutic effect. The exact amount required will vary from subject to subject depending on factors such as the species being treated, the severity of the halitosis, the particular agent being administered, etc. Thus, whilst it is not possible to specify an exact "effective amount", for any given case, an appropriate "effective amount" may be determined by one of ordinary skill in the art using only routine experimentation.

### Description of the Figures

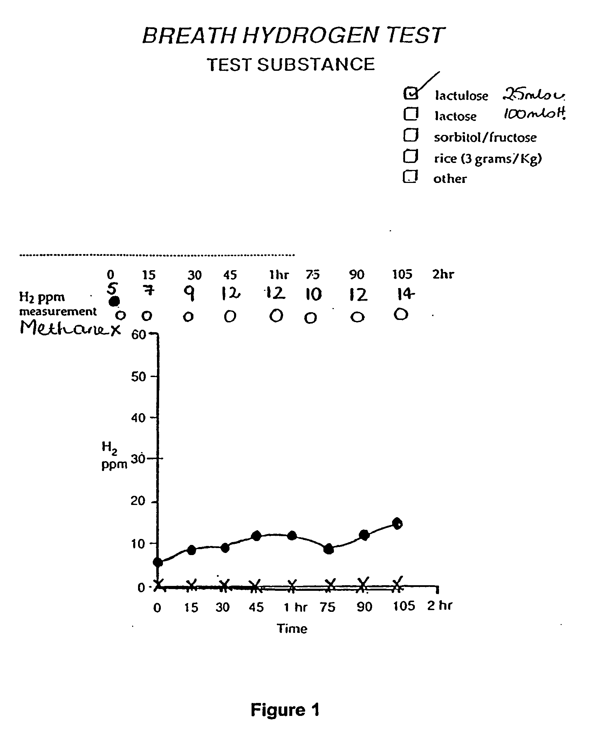

Figure 1 shows the results of a breath hydrogen test on a human subject suffering from halitosis.

### Description of the Invention

It has been found that administration of a bismuth salt or bismuth containing composition to a vertebrate suffering halitosis is an effective way of reducing or substantially eliminating malodorous breath. In general, the subject being treated is not suffering from *H. pylori* infection.

A large proportion of intestinal gas is reabsorbed from the colon and expired in the breath. This is usually without aroma, particularly in individuals (approximately half of the population) who have primarily methanogenic bacteria within their colon which produce hydrogen, carbon dioxide and methane. The remaining half of the population have a predominantly sulfite-reducing bacterial population in their colon and the principal gases produced are odorous volatile sulfur compounds, such as hydrogen sulfide, methyl mercaptan and dimethyl sulfide.

The present invention is related to the realisation that in normal subjects not suffering from *H. pylori* infection, and whose colons predominantly contain a sulfite-reducing bacterial population, the sulfite-reducing bacteria may be the dominant source of malodorous gases.

Recent studies involving quantitative measurement of volatile sulfide compounds obtained from saliva using the Halimeter™ sulfide monitor (available from Interscan Corporation, PO Box 2496, Chatsworth California 91313-2496 USA), identified the predominant compounds present were hydrogen sulfide, methanethiol and dimethyl sulfide (lerardi E., et al. Halitosis and Helicobacter pylori - a possible relationship. Dig. Dis. Sci.1998;43:2733-2737). All of these gases are primarily formed within both the mouth and the colon by bacterial fermentation.

Oral administration of a bismuth salt or a bismuth containing composition enables bismuth to bind sulfurous gases in the gut, thereby substantially preventing the absorption of such malodorous gases from the colon and reducing their subsequent excretion in the breath. Oral administration of a bismuth salt or a bismuth containing compound can also reduce the concentration of odorous gases (of gut or oral origin) within the oral cavity.

Preferably, ingestion of a therapeutically effective amount of bismuth does not eliminate the hydrogen sulfide-producing bacterial population in the gut of the vertebrate being treated, rather, the bismuth is able to bind to odorous gases produced by such bacteria, thereby substantially preventing expiration of such gases in the breath.

### 1. Breath Analysis

Typically, a subject undergoes breath analysis to assess the extent of halitosis (eg, using the Halimeter™ breath test) and/or to determine the nature of the colonic bacterial population (eg, using the lactulose breath test). Methanogenic and sulfite-reducing bacterial populations are generally mutually exclusive. The presence of methane in the breath analysis indicates the subject does not have a dominant sulfite-reducing bacterial population. Breath analysis is typically carried out prior to and/or during treatment of halitosis.

### 1.1 The Halimeter™ Test

The Halimeter™ sulfide monitor can be used to assess halitosis by detecting the presence of volatile hydrogen sulfide gas and to determine whether hydrogen sulfide gas is the major gas in the breath emission of a subject. Production of hydrogen sulfide without concomitant production of methane is an indication that the subject has a dominant sulfite-reducing bacterial population in the gut.

### 1.2. Lactulose Breath Test

Determining whether a subject with halitosis has dominant sulfite-reducing bacterial population in the gut can be achieved using a lactulose breath test, which involves administering a synthetic disaccharide (lactulose) to a subject. Generally, the lactulose is provided in solution form for the subject to drink. Breath samples are collected at intervals (typically by having the subject breathe into a small balloon) and analysed for the presence of methane and hydrogen, eg, using the Quintron microlyser (Endomed Pty Ltd, PO Box 455, Strathpine Qld 4500, Australia). The presence of methane as a major component of the breath indicates the presence of a methanogenic bacterial population in the gut. Detection of hydrogen gas alone (ie, substantially no methane detected) as the major gas component in exhaled breath indicates of a sulfite-reducing bacterial gut population.

Typically, a ¹³C urea breath test can be carried out concurrently with the lactulose breath test in order to determine whether the vertebrate suffers from *Helicobacter pylori* infection in the gut, which is a possible causative factor of halitosis. If the subject is found to be suffering from *H. pylori* infection, they can be treated using known therapies, eg, triple therapy (comprising administration of a proton pump inhibitor (eg, omeprazole), in combination with clarithromycin and amoxycillin or tetracycline). Typically, if symptoms of halitosis persist after eradication of *H. pylori,* the halitosis is treated in accordance with the present invention.

### 2. Treatment of Halitosis

Patients exhibiting halitosis are administered an oral dose of a bismuth salt or a bismuth containing pharmaceutical composition. Generally, the bismuth salt or bismuth containing composition is administered in a form suitable to ensure the bismuth component reaches the gut where it is able to bind to sulfurous gases, thereby reducing or substantially preventing excretion of such gases in the breath. Examples of suitable formulations for enabling the bismuth to reach the gut include but are not limited to, liquids, suspensions, emulsions, non-chewable tablets and capsules. Oral administration of such formulations can also reduce the concentration of odorous gases (of gut or oral origin) within the oral cavity.

Further, the vertebrate may be administered an oral dosage form of a bismuth salt or a bismuth containing composition that reduces or substantially eradicates malodorous breath (ie, halitosis) within the oral cavity, irrespective of whether the malodorous gases are primarily of oral or gut origin. Examples of suitable formulations include but are not limited to bismuth containing mouth washes, chewing gum, toothpaste, lozenges, chewable tablets and capsules. Such dosage formulations may be administered concomitantly with, separately from, or in addition to the above dosage forms which are designed to reach the gut.

Typical bismuth salts suitable for use in the present invention include but are not limited to bismuth subcitrate, aluminate, oxide, salicylate, subgallate, tannate, phosphate, tribromphenate, subcarbonate, subnitrate, and mixtures thereof. Examples of bismuth containing pharmaceutical compositions suitable for use in the present invention include but are not limited to De-nol™ (active ingredient bismuth subcitrate) and Pepto-bismol® (active ingredient bismuth subcitrate).

More typically, the bisumuth salt suitable for use in the present invention is selected from bismuth subcitrate and bismuth salicylate.

Single or multiple administrations of bismuth-containing compositions can be carried out with dose levels and pattern being selected by the treating physician. The compositions useful in of the present invention should provide a quantity of the bismuth salt sufficient to effectively treat the vertebrate.

One skilled in the art would be able, by routine experimentation, to determine an effective, non-toxic amount of the bismuth salts and bismuth-containing pharmaceutical compositions useful in the present invention which would be required to effectively treat halitosis. Generally, an effective dosage is expected to be in the range of about 0.0001 mg to about 1000mg per kg body weight per 24 hours; more typically, about 0.001 mg to about 750mg per kg body weight per 24 hours; even more typically about 0.01 mg to about 500mg per kg body weight per 24 hours; even more typically about 0.1 mg to about 500mg per kg body weight per 24 hours; even more typically about 0.1mg to about 250mg per kg body weight per 24 hours; even more typically about 1.0mg to about 250mg per kg body weight per 24 hours. More typically, an effective dose range is expected to be in the range about 1.0mg to about 200mg per kg body weight per 24 hours; more typically about 1.0mg to about 100mg per kg body weight per 24 hours; even more typically about 1.0mg to about 50mg per kg body weight per 24 hours; even more typically about 1.0mg to about 25mg per kg body weight per 24 hours; even more typically about 5.0mg to about 50mg per kg body weight per 24 hours; even more typically about 5.0mg to about 20mg per kg body weight per 24 hours; even more typically about 5.0mg to about 15mg per kg body weight per 24 hours.

A typical treatment regime for a human exhibiting symptoms of halitosis will involve administering bismuth in a suitable dosage form three times daily, more typically twice daily, even more typically once daily, until the symptoms of halitosis are eradicated, and thereafter at the same or reduced dosage rate for a suitable period. Still typically, the bismuth salt is not administered on a daily basis. Even more typically, administration of bismuth may cease for a period of during a course of treatment.

Typically, suitable treatment for a human subject includes administering bismuth subcitrate or bismuth subsalicylate twice daily, more typically once daily.

Typically, a suitable dosage rate is between 10mg to 400 mg/day; more typically 20mg to 150 mg/day, even more typically 50mg to 100mg/day.

Typically the treatment would be for the duration of the condition. Typically, the duration of the treatment is between 2 days and 8 weeks; more typically, between 1 week and 6 weeks; still typically between 2 weeks and 5 weeks; even more typically between 3 and 4 weeks; yet even more typically, 4 weeks. Still typically the duration of treatment is for 1 to 2 weeks.

Further, it will be apparent to one of ordinary skill in the art that the optimal quantity and spacing of individual dosages of a bismuth salt or bismuth-containing composition used in the present invention will be determined by the nature and extent of the condition being treated, the form, route and site of administration, and the nature of the particular vertebrate being treated. Also, such optimum regimes can be determined by conventional techniques.

It will also be apparent to one of ordinary skill in the art that the optimal course of treatment, such as, the number of doses of the compound given per day for a defined number of days, can be ascertained by those skilled in the art using conventional course of treatment determination tests. If necessary, the treatment regime can be repeated periodically to treat recurring halitosis. The dosage rates of bismuth may be the same or may vary throughout the treatment.

Whilst the bismuth salts useful in the present invention may be administered alone, it is generally preferable that the compound be administered as a pharmaceutical composition/formulation. In general, pharmaceutical formulations may be prepared according to methods which are known to those of ordinary skill in the art and accordingly may include a pharmaceutically acceptable carrier, diluent and/or adjuvant.

The carriers, diluents and adjuvants must be "acceptable" in terms of being compatible with the other ingredients of the formulation, and not deleterious to the recipient thereof.

Examples of pharmaceutically and veterinarily acceptable carriers or diluents are demineralised or distilled water; saline solution; vegetable based oils such as peanut oil, safflower oil, olive oil, cottonseed oil, maize oil, sesame oils such as peanut oil, safflower oil, olive oil, cottonseed oil, maize oil, sesame oil, arachis oil or coconut oil; silicone oils, including polysiloxanes, such as methyl polysiloxane, phenyl polysiloxane and methylphenyl polysiloxane; volatile silicones; mineral oils such as liquid paraffin, soft paraffin or squalane; cellulose derivatives such as methyl cellulose, ethyl cellulose, carboxymethylcellulose, sodium carboxymethylcellulose or hydroxypropylmethyl-cellulose; lower alkanols, for example ethanol or iso-propanol; lower aralkanols; lower polyalkylene glycols or lower alkylene glycols, for example polyethylene glycol, polypropylene glycol, ethylene glycol, propylene glycol, 1,3-butylene glycol or glycerin; fatty acid esters such as isopropyl palmitate, isopropyl myristate or ethyl oleate; polyvinylpyrrolidone; agar; carrageenan; gum tragacanth or gum acacia, and petroleum jelly. Typically, the carrier or carriers will form from 10% to 99.9% by weight of the compositions.

The bismuth salts and bismuth-containing compositions useful in the present invention may be administered by standard routes. In general, the salts and compositions may be administered orally. Still generally, the compositions may be in the form of a capsule, tablet, lozenge, suspension or solution suitable for oral ingestion, or in an aerosol form suitable for administration by oral inhalation, or in the form of an oral mouthwash, gum or tooth paste preparation. In addition these oral formulations may contain suitable flavouring and colourings agents. When used in capsule form the capsules may be coated with compounds such as glyceryl monostearate or glyceryl distearate which delay disintegration of the capsule.

Adjuvants typically include emollients, emulsifiers, thickening agents, preservatives, bactericides and buffering agents.

Solid forms for oral administration may contain binders acceptable in human and veterinary pharmaceutical practice, sweeteners, disintegrating agents, diluents, flavourings, coating agents, preservatives, lubricants and/or time delay agents. Suitable binders include gum acacia, gelatine, com starch, gum tragacanth, sodium alginate, carboxymethylcellulose or polyethylene glycol. Suitable sweeteners include sucrose, lactose, glucose, aspartame or saccharine. Suitable disintegrating agents include com starch, methylcellulose, polyvinylpyrrolidone, guar gum, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulfite. Suitable lubricants include magnesium stearate, stearic acid, sodium oleate, sodium chloride or talc. Suitable time delay agents include glyceryl monostearate or glyceryl distearate.

Liquid forms for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides, or mixtures thereof.

Suspensions for oral administration may further comprise dispersing agents and/or suspending agents. Suitable suspending agents include sodium carboxymethylcellulose, methylcellulose, hydroxypropylmethyl-cellulose, polyvinylpyrrolidone, sodium alginate or acetyl alcohol. Suitable dispersing agents include lecithin, polyoxyethylene esters of fatty acids such as stearic acid, polyoxyethylene sorbitol mono- or di-oleate, -stearate or -laurate, polyoxyethylene sorbitan mono- or di-oleate, -stearate or-laurate, and the like.

The emulsions for oral administration may further comprise one or more emulsifying agents. Suitable emulsifying agents include dispersing agents as exemplified above, or natural gums such as guar gum, gum acacia or gum tragacanth.

The invention will now be described in greater detail by reference to specific Examples which should not be construed as in any way limiting the scope of the invention.

### Example 1

A breath analysis study was conducted on eight human subjects each exhibiting halitosis. The Halimeter™ was used to test for the presence of volatile hydrogen sulfide gas and to determine whether hydrogen sulfide gas was the major gas in the breath emission of each subject. The Halimeter™ readings indicated each subject produced hydrogen sulfide however all failed to produce methane. This result indicated that the tested subjects had a dominant sulfite-reducing bacterial population in the gut capable of producing hydrogen sulfide gas. The results of the breath analysis test for one human subject are shown in Figure 1.

### Example 2

Eight subjects exhibiting halitosis and having a dominant sulfite-reducing bacterial population in the gut as evidenced by the Halimeter™ according to Example 1, were administered a 120mg oral dose of De-nol™ three times daily for a period of 4 weeks. The eight subjects treated exhibited symptomatic improvement of halitosis within 3-7 days.

### Example 3

A lactulose breath analysis was performed on 25 human subjects exhibiting symptoms of halitosis. The test confirmed the absence of methane, which was indicative of a dominant sulfite reducing bacterial population in the colon. All 25 subjects were treated with De-nol™ (120mg, tds) and all 25 subjects exhibited subjective symptomatic improvement of their halitosis within 2-7 days.

### Example 4

De-nol™ was administered in tablet form (120mg) to 20 subjects exhibiting symptoms of halitosis at a dose rate of two tablets per day until the symptoms were alleviated. Once the symptoms of halitosis were eradicated, subjects were then administered 1 tablet of De-nol™ per day for 14 days, which was sufficient to maintain alleviation of symptoms of halitosis.

It will be appreciated that the present invention has been described with respect to preferred embodiments and various modifications and improvements can be made without deviating from the spirit and scope of the invention.

## Claims

1. Use of a therapeutically effective amount of a bismuth salt or bismuth containing composition in the manufacture of a medicament for treating halitosis in a vertebrate in need of said treatment wherein said halitosis is associated with sulfite-reducing bacteria in the gut.

2. Use according to claim 1, wherein a therapeutically effective amount of said medicament is administered to said vertebrate when a sulfite reducing bacterial population is detected in the gut.

3. Use according to claim 1 or 2, wherein said vertebrate does not suffer from *Helicobacter pylori* infection.

4. Use according to claim 1 or 2, wherein the bismuth salt is selected from the group consisting of bismuth subcitrate, bismuth aluminate, bismuth oxide, bismuth salicylate, bismuth subgallate, bismuth stannate, bismuth phosphate, bismuth tribromphenate, bismuth subcarbonate, bismuth subnitrate, bismuth sodium tartrate, and mixtures thereof.

5. Use according to claim 4, wherein the bismuth salt is selected from the group consisting of bismuth salicylate and bismuth subcitrate.

6. Use according to claim 4, wherein said bismuth salt is administered orally.

7. Use according to claim 1 or 2, wherein said vertebrate is a human.

8. Use according to claim 1; wherein the medicament is formulated as a delayed-release capsule.

9. Use according to claim 1, wherein the sulfite-reducing bacteria are in the colon.

## Patentansprüche

1. Verwendung einer therapeutisch wirksamen Menge eines Bismutsalzes oder einer Bismut enthaltenden Zusammensetzung bei der Herstellung eines Medikaments zur Behandlung von Halitosis bei einem Vertebraten, der der Behandlung bedarf, wobei die Halitosis mit Sulfit-reduzierenden Bakterien im Darm verbunden ist.

2. Verwendung gemäß Anspruch 1, wobei eine therapeutisch wirksame Menge des Medikaments an den Vertebraten verabreicht wird, wenn eine Sulfit-reduzierende Bakterienpopulation im Darm detektiert wird.

3. Verwendung gemäß Anspruch 1 oder 2, wobei der Vertebrat nicht an *Helicobacter pylori-*Infektion leidet.

4. Verwendung gemäß Anspruch 1 oder 2, wobei das Bismutsalz ausgewählt ist aus der Gruppe, bestehend aus Bismutsubcitrat, Bismutaluminat, Bismutoxid, Bismutsalicylat, Bismutsubgallat, Bismutstannat, Bismutphosphat, Bismuttribromphenat, Bismutsubcarbonat, Bismutsubnitrat, Bismutnatriumtartrat und Gemischen davon.

5. Verwendung gemäß Anspruch 4, wobei das Bismutsalz ausgewählt ist aus der Gruppe, bestehend aus Bismutsalicylat und Bismutsubcitrat.

6. Verwendung gemäß Anspruch 4, wobei das Bismutsalz oral verabreicht wird.

7. Verwendung gemäß Anspruch 1 oder 2, wobei der Vertebrat ein Mensch ist.

8. Verwendung gemäß Anspruch 1, wobei das Medikament als Kapsel mit verzögerter Freisetzung formuliert ist.

9. Verwendung gemäß Anspruch 1, wobei die Sulfit-reduzierenden Bakterien im Colon sind.

## Revendications

1. Utilisation d'une quantité thérapeutiquement efficace d'une composition contenant un sel de bismuth ou du bismuth dans la fabrication d'un médicament destiné au traitement de l'halitose chez un vertébré ayant besoin dudit traitement où ladite halitose est associée à des bactéries sulfito-réductrices dans l'intestin.

2. Utilisation selon la revendication 1, où une quantité thérapeutiquement efficace dudit médicament est administrée au dit vertébré lorsqu'une population de bactéries sulfito-réductrices est détectée dans l'intestin.

3. Utilisation selon la revendication 1 ou 2, où ledit vertébré ne souffre pas d'une infection à *Helicobacter pylori.*

4. Utilisation selon la revendication 1 ou 2, où le sel de bismuth est choisi dans le groupe constitué du sous-citrate de bismuth, de l'aluminate de bismuth, de l'oxyde de bismuth, du salicylate de bismuth, du sous-gallate de bismuth, du stannate de bismuth, du phosphate de bismuth, du tribromophénate de bismuth, du sous-carbonate de bismuth, du sous-nitrate de bismuth, du tartrate de bismuth et de sodium, du et des mélanges de ceux-ci.

5. Utilisation selon la revendication 4, où le sel de bismuth est choisi dans le groupe constitué du salicylate de bismuth et du sous-citrate de bismuth.

6. Utilisation selon la revendication 4, où ledit sel de bismuth est administré par voie orale.

7. Utilisation selon la revendication 1 ou 2, où ledit vertébré est un être humain.

8. Utilisation selon la revendication 1, où le médicament est formulé sous la forme d'une capsule à libération retardée.

9. Utilisation selon la revendication 1, où les bactéries sulfito-réductrices sont dans le côlon.
